# EUROPEAN PATENT APPLICATION

(11) **EP 4 424 809 A1**
(43) Date of publication of application: **04.09.2024**
(21) Application number: 23382198.2
(22) Date of filing: 03.03.2023
(51) Int. Cl.: C12M 1/00, C12P 17/12, C12P 39/00, C12M 1/02, C12N 1/38

(54) **METHOD FOR CONVERTING METHANE-CONTAINING GAS STREAMS INTO OSMOLYTES USING A BACTERIA CULTURE IN A TAYLOR-FLOW BIOREACTOR**

(71) Applicant: FCC Aqualia, S.A., 28050 Madrid (ES)
(72) Inventor: RODERO RAYA, María del Rosario, 47011 Valladolid (ES); PEREZ MARTINEZ, Víctor, 47011 Valladolid (ES); MUÑOZ TORRE, Raúl, 47011 Valladolid (ES); LEBRERO FERNANDEZ, Raquel, 47011 Valladolid (ES); CANTERA RUIZ-DE- PELLÓN, Sara, 47011 Valladolid (ES); ZAMORA BONACHELA, Patricia, 28050 Madrid (ES); ROGALLA, Frank, 28050 Madrid (ES); MONSALVO GARCÍA, Víctor, 28050 Madrid (ES)
(74) Representative: Ungria López, Javier

(57) **Abstract**

A method for bioconversion of methane-laden gas streams (e.g. natural gas, biogas or diluted methane emissions) into high added-value products such as ectoine and hydroxyectoine, in high-mass transfer bioreactors, such as a Taylor-flow bioreactor, as well as the use of said bioreactors for biological bioconversion processes wherein a microorganism is used for the production of osmolytes (e.g. ectoine, hydroxyectoine or any derivative thereof).

## Description

### FIELD OF THE INVENTION

The present invention relates to a method for bioconversion of methane-laden gas streams (e.g. natural gas, biogas or diluted methane emissions) into high added-value products of industrial interest such as ectoine and hydroxyectoine, in high-mass transfer bioreactors, such as a Taylor-flow bioreactors, as well as the use of said bioreactors for biological bioconversion processes wherein a microorganism is used for the production of osmolytes (e.g. ectoine, hydroxyectoine or any derivative thereof). Therefore, the invention pertains to the field of the waste management, biochemical, biotechnological, medical, cosmetic and pharmaceutical industries.

### BACKGROUND OF THE INVENTION

Methanotrophic bacteria are aerobic microorganisms with the ability to use methane as their sole carbon and energy source. Particularly, haloalkalophilic methanotrophic bacteria are characterised by a high resistance towards high salinity and pH conditions in the environment. This bacterial resistance is determined by the ability to produce high amounts of intracellular ectoine and hydroxyectoine during methane oxidation under high saline stress.

Ectoine, hydroxyectoine or any derivative thereof are compatible solutes that provide a high osmotic resistance to the bacterial cells. Both compounds act as osmotic protective agents, and they are also excellent biofunctional stabilisers, skin protectors and potential drugs for the treatment of diseases such as Alzheimer's and rhinoconjunctivitis. They are used as components of anti-wrinkle and moisturising cosmetics, among other wide possibilities of their application in biotechnology, cosmetology, medicine and pharmacy.

Haloalkalophilic methanotrophic bacteria are able to excrete the intracellular ectoine, hydroxyectoine and any derivative thereof when subjected to a rapid decrease in the salinity conditions, known as hypo-osmotic shock. This bacterial ability of producing and excreting these bioproducts without suffering from cellular damage has an industrial interest for the biochemical, biotechnological, medical, cosmetic and pharmaceutical industries, since it allows the continuous harvesting of bioproducts (ectoine, hydroxyectoine and any derivative thereof) as well as the re-utilization of bacterial biomass in batch, fed-batch and continuous production cycles. This overall process is defined as bacterial biomilking.

The opportunity of transforming methane-laden streams, such as natural gas, biogas from the anaerobic digestion of organic waste or diluted methane emissions from industrial activities, into high added-value products, has emerged as a profitable gas valorisation route in all sorts of waste management and waste bioconversion facilities.

However, gas fermentation technologies are usually constrained by the limited gas-liquid mass transfer of certain hydrophobic compounds such as methane and oxygen. These processes have been typically carried out in bubble column bioreactors or continuous stirred tank bioreactors to increase the gas-liquid mass transfer of hydrophobic gas compounds, which normally entail high capital and operational costs.

This is the case of reference US2021403859A1, which discloses a method of enhancing productivity of the biomass and the value added products such as ectoine by conversion of gaseous substrate such as methane in a stirred tank reactor using starter microorganism selected from a broad group, such as *M. capsulatus.* Derived from said disclosure, one expert would see that the C1 conversion efficiency with stirrer tank bioreactors is low and energetically very intensive, and no assessment on the recovery of ectoine, hydroxyectoine or any derivative thereof from the bacterial biomass is analysed. Process performance in this document requires the consumption of pH control agents, and the operational costs are high.

In addition, Cantera et al. 2018 (Review. Technologies for the bioconversion of methane into more valuable products), discloses the bioconversion of methane into microbial molecules with a high retail value in the industry, such as ectoine, feed proteins, biofuels, bioplastics and polysaccharides, apart from new bioreactors recently tested in discontinuous and continuous mode operation, such as a modified airlift bioreactor. Biomilking is commented on this work, but without using methanotrophic bacteria. The use of industry by-products containing methane (e.g., biogas, natural gas) was not evaluated in this study. The economic study herein presented did not include the use of innovative device/steps that minimize and optimize the costs.

Besides, Cantera et al. (2019. Novel haloalkaliphilic methanotrophic bacteria: an attempt for enhancing methane biorefinery) focuses on the production of different ectoines during CH₄ biodegradation. Different enrichments were carried out in basal media supplemented with 3 and 6 % NaCl, and using methane as the sole carbon and energy source. The amount of ectoine accumulation was analysed, as well as the production of hydroxyectoine using methane as a feedstock. Two novel strains (representing novel species) capable of using methane as the sole carbon and energy source were isolated: *Alishewanella sp.* strain RM1 and *Halomonas sp.* strain PGE1. This study does not include the evaluation of the continuous production of ectoines in bioreactors nor the subsequent biomilking process for ectoine recovery from the bacterial biomass cells. The use of industrial methane-laden gas streams such as biogas or natural gas is not evaluated.

Some other documents, such as US20210070810A1, WO2022173436A1, CN114807263A, and US20160237442A1 disclose different processes for the production of ectoines. These documents do not analyse the use of alternative devices, such as bioreactors, or raw methane-containing streams which may enhance the bioconversion process. These documents do not include the potential utilisation of industrial emissions containing methane.

Taking into account the drawbacks observed in the prior art, there is a need in the gas fermentation field of applying innovative strategies and bioreactors configuration to boost the gas-liquid mass transfer of poorly soluble compounds. The improvement of the current poor process performance would entail a significant enhancement of methane fermentation cost-efficiency and therefore a reduction of the production costs of added-value products.

This is the object of the present invention, by using high-mass transfer bioreactors for the cultivation of bacteria that transform a flow of gas comprising methane into osmolytes (e.g. ectoine, hydroxyectoine or any derivative thereof). One of these high-mass transfer bioreactors is the Taylor-flow bioreactor.

### DESCRIPTION OF THE INVENTION

The present invention is firstly directed to a biomilking method for converting methane-laden gas streams into added-value products (such as ectoine and derivatives thereof -hydroxyectoine- or any osmoprotective osmolyte) through a biological process using extremophile methanotrophic bacteria in a high-mass transfer bioreactor, according to claim 1. The biomilking method comprises:
a) cultivating an extremophile methanotrophic bacteria culture in a nutrient liquid solution comprising a content of salt of at least 0.05% w/w, more preferably between 1%w/w and 36%w/w (6%w/w in the most preferred case); the nutrient liquid solution being one selected from the group consisting of: a synthetic solution of mineral salts, a waste stream and a mixture thereof (including a mixture of different waste streams);
b) injecting a gas flow containing methane and oxygen, in such a way that the flow of gas comprises between 1%v/v and 99%v/v of methane and between 1%v/v and 99%v/v of oxygen; the ratio of oxygen-to-methane being of at least 1.5 mol oxygen/mol methane;
c) extracting a fraction of the bacteria culture and subjecting to a concentration process of the biomass between 10 and 400 (preferably up to 200) g biomass / L in a solid liquid separator (e.g. centrifuge);
d) subjecting the concentrated biomass to a hypo-osmotic shock during a period of time comprised between 1 minute and 24 hours (preferably up to 60 minutes) in an external tank by adding a liquid stream, which is water, with a salinity ranging from 0 %w/w to 6%w/w, lower than the one of the concentrated biomass;
e) concentrating again the biomass after the hypo-osmotic shock to a concentration between 10 and 400 g biomass /L (preferably between 10 and 200 g biomass /L) in a solid liquid separator (e.g. centrifuge); and
f) extracting one or more osmoprotective osmolytes;

wherein the biomass is re-used by one of the methods selected from the group consisting of batch, fed-batch or continuous production cycles for obtaining a liquid stream containing osmolytes (e.g. ectoine, hydroxyectoine or any derivative thereof); and
wherein all or part of the used gas stream that leaves the bioreactor is recirculated by re-injecting said used gas stream into the bioreactor, separately or mixed with the gas flow containing methane and oxygen.

In brief, the extremophile methanotrophic bacteria culture is cultivated inside the bioreactor creating a high salinity environment for fostering the bioproduction and accumulation of osmoprotective osmolytes inside the bacterial cells; said osmolytes are in the most preferred case ectoine, hydroxyectoine or any derivative thereof. Part of said culture is subsequently subjected to a hypo-osmotic shock using water with a salinity range lower than that of the original culture, causing the partial release of at least one osmoprotective osmolyte from the bacterial cells. To add more, once the osmolytes are obtained and extracted it is possible to re-use the biomass again (after concentration) because the bacteria do not suffer any cellular damage from the earlier hypo-osmotic shock.

All these stages and the production of osmolytes are possible thanks to the use of a high-mass transfer bioreactor, unlike the processes known in the prior art. For instance, US20210403859A1 uses stirred reactors. In the most preferred case, the selected high-mass transfer bioreactor is a Taylor-flow bioreactor. This type of reactor comprises a mixing chamber wherein the gas and the liquid phase come into contact prior to flow up through multiple capillary tubes and reached the upper chamber for their separation; wherein the capillaries allow a segmented flow of gas and liquid bubbles, where capillary forces are dominant.

Taylor-flow bioreactors have been typically used for catalysis and hydrogenation applications but never for the production of osmolytes (e.g. ectoine, hydroxyectoine or any derivative thereof), unlike the present invention. It has been observed that the configuration of these compact bioreactors allows for an unexpected enhancement in gas-liquid mass transfer while avoiding excessive reactor volumes and pressure drops, thus reducing significantly the capital and operational costs.

In a preferred embodiment, the extremophile methanotrophic bacteria are selected among haloalkalophilic methanotrophic bacteria or halophilic methanotrophic bacteria. The bacteria culture can be either a pure culture or a mixed culture.

The nutrient liquid solution used for growing the bacteria culture in step a) can be preferably a by-product of the industry, for instance a waste stream. This is an advantageous solution, because the state of the art only reported the use of synthetic mixture of salts or trace elements that supports and enhances methanotrophic growth, but not the use of waste streams. In a preferred embodiment, the water stream can be supplemented with at least one trace metal. The trace metal/s can be selected from the group consisting of cupper, iron, zinc, nickel, cobalt, molybdenum, manganese, boric acid or any combination thereof. Preferably, the waste water is supplemented with a trace metal solution containing cupper, iron, zinc, nickel, cobalt, molybdenum, manganese, boric acid or any combination thereof. When the waste stream is a brine wastewater, which is one of the most preferred cases, said brine wastewater must be supplemented as explained with a trace metal solution.

In a particular embodiment, the waste stream (for instance, a brine wastewater supplemented with trace metals) can be mixed with a synthetic stream. In another, more preferred embodiment, the nutrient liquid solution is a mixture of two or more waste streams, without synthetic mixtures. This case is more preferred because it is observed that the growth rate of the bacteria culture and the final production of osmoprotective osmolytes are significantly higher than those registered when only synthetic streams of salts are used. The use of waste streams as nutrient solutions enhances the whole biomilking process, for different reasons: lowering the operating costs of the industrial process, providing a circular economy for waste stream management, and overall, obtaining a more efficient process for bacterial growth. Some evidences are provided below.

The gas flow or stream containing methane and oxygen injected in step b) may be in a preferred case a by-product of the industry, such as for instance biogas or diluted methane waste emissions. This is the most advantageous case, because the raw material is a low-value by-product extracted from the industry. Said flow of methane-laden stream usually comprises an amount of at least 1%v/v of methane of the total flow of gas, and said amount is equal or lower than 99%v/v of the total gas flow, as imposed in the present invention.

In one particular embodiment, only one gas stream containing methane and oxygen is injected in step b), in such a way that the flow of gas may be a single gas stream from its origin. Alternatively, the gas flow may be a mixture of a methane-laden gas stream (e.g. natural gas, biogas or diluted methane emissions) and an oxygen-laden gas stream (e.g. pure oxygen, enriched air or atmospheric air) in only one flow. In another particular embodiment, the gas flow may be made of more than one gas stream having different origins, which are injected separately. For instance, the above-mentioned methane-laden gas stream and the oxygen-laden gas stream can be also injected separately, always resulting in the concentrations given above, and presenting an oxygen-to-methane ratio of at least 1.5 mol oxygen/mol methane. In a preferred case, the amount of methane is of 5% in air (for instance, resulting from the mixture of biogas or natural gas with air or O₂ enriched air, which may be injected as a mixture or separately).

Preferably, the ratio of oxygen-to-methane is from 1.5 mol oxygen/mol methane to an amount of 10 mol oxygen/mol methane. In the most preferred case, the ratio is 1.5 mol oxygen/mol methane.

All or part of the gas flow that has been used in the process and that is discarded from (i.e. that leaves) the bioreactor is also recirculated from the gas stream outlet to the gas flow inlet of the bioreactor, thus enhancing the recovery rate of the whole process. The internal gas recirculation, which is injected into the bioreactor either previously mixed with the inlet gas flow or separately, aims at increasing the gas residence time of methane in order to increase its conversion efficiency. Preferably, the fraction of the gas stream outlet that is recirculated with the gas flow inlet into the bioreactor varies between 90% and 99% of the total gas stream outlet.

The fraction of bacteria culture extracted in step c) is approximately between 5% and 100% of the total working volume of the bioreactor per day. In the most preferred case, the fraction of bacterial culture extracted is 20% of the total working volume of the bioreactor per day.

A liquid waste stream is also produced in step c) as a result of the concentration stage, and one of the additional advantages of the process of the invention in that said waste stream can be recycled to the high mass transfer bioreactor.

The injection of the liquid stream into the high-mass transfer bioreactor in step d) can be performed in batch, fed-batch or continuous operation cycles.

Besides, the harvesting of the biomass in step d) from the high-mass transfer bioreactor to the hypo-osmotic tank can be also performed in batch, fed-batch or continuous operation cycles.

According to the above, it is a second object of the invention the use of a high-mass transfer bioreactor, preferably a Taylor-flow bioreactor, for the bioconversion of methane-containing streams into added-value products (such as the osmoprotective osmolytes cited above) by biological processes, such as biomilking processes.

To sum up, the present invention discloses the following improvements and goals in the field:
- The use of high-mass transfer bioreactors, such as Taylor-flow bioreactors for the production of ectoine, hydroxyectoine or any osmoprotective osmolyte.
- The use of extremophile methanotrophic bacteria for the transformation of gas streams comprising methane into the above-mentioned added-value products.
- The use of salt-rich waste streams for increasing the salinity of the cultivation broth, thus fostering ectoine and hydroxyectoine accumulation, in Taylor Flow bioreactors.

The installation for carrying out the claim process is also an object of the present invention, and comprises: a high mass transfer bioreactor, for the bioconversion of methane into the desired products; connected to a solid-liquid separator for concentrating the bacterial biomass; a stirred tank reactor or vessel for performing the hypo-osmotic shock (also named as hypo-osmotic tank) and release the bioproduct; and a solid-liquid separator for recovering the bioproduct and recyclying the bacterial biomass to the high mass-transfer bioreactor.

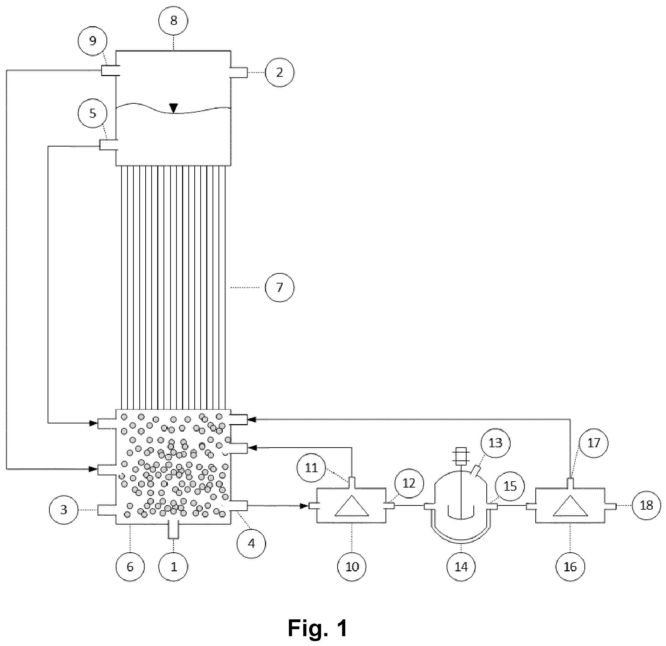

The high mass-transfer bioreactor comprises the main elements 6, 7 and 8 shown in Figure 1, i.e. a gas-liquid mixing chamber (6), wherein the bacterial culture is mixed with the gas flow entering the reactor (both the fresh fed and internal gas recirculation, see below) below a group of Taylor flow capillaries (7) which are connected at the other end to a gas-liquid separator (8). The gas-liquid mixing chamber (6) is located at the bottom part of the bioreactor, wherein the methane- and oxygen-laden stream or streams enters the chamber through the gas inlet (1), and is/are mixed with the high salinity liquid stream that enters the chamber (6) through the liquid inlet (3) and the pipeline system (5) for liquid recirculation, for cultivating the bacteria. The high gas-liquid mass transfer is performed in a series of thin capillaries (7), which are operated under Taylor flow. These capillaries (7) connect the bottom part of the bioreactor, i.e. the gas-liquid mixing chamber (6) and the upper part, i.e. the gas-liquid separator (8). The biphasic upstream flow in these capillaries (7) is performed as a bubble train or Taylor-flow characterised by an alternate flow of small bubbles of gas and drops of liquid. The upper part of the bioreactor is a gas-liquid separator (8) or separation chamber in which gas and liquid are separated. The gas stream leaves the high mass-transfer bioreactor through an outlet (2) located at the gas-liquid separator (8) and can be either vented or flared. The liquid stream that leaves the gas-liquid separator (8) is recycled to the gas-liquid mixing chamber (6) through a pipeline system (5). Additionally, a fraction of the outlet gas stream produced in the gas-liquid separator (8) can be recycled through a gas recirculation pipeline system (9) and mixed with the raw gas stream in the gas-liquid mixing chamber (6) to increase the gas residence time of methane in the bioreactor.

Once the biomass has grown inside the bioreactor, a fraction of the bacterial culture is harvested through an outlet (4) at the gas-liquid mixing chamber (6) with a liquid stream and the extracted biomass is concentrated in a solid-liquid separator (10). The liquid stream (11) obtained from the solid-liquid separator (10) can be recycled to the bottom part of the high mass transfer bioreactor, i.e. at the gas-liquid mixing chamber (6), through a pipeline system (11).

The concentrated biomass stream that is solid, obtained at the solid-liquid separator (10) leaves said solid-liquid separator (10) through another outlet (12) and is fed to a stirred tank bioreactor, which has the function of a hypo-osmotic shock vessel or tank (14). A water or a lower salinity medium stream is fed to the hypo-osmotic shock vessel (14) through an inlet (13) in order to decrease the salinity and induce a hypo-osmotic shock that will result in the release of the bioproducts from the cells, i.e. osmolytes (e.g. ectoine, hydroxyectoine or any derivative thereof). The resulting liquid stream leaves the hypo-osmotic shock vessel (14) through an outlet (15) and is subsequently concentrated in a second solid-liquid separator (16). The liquid stream obtained at the second solid-liquid separator (16) contains the bioproducts to be purified and is extracted through the outlet (18). The stream of concentrated biomass obtained from the solid-liquid separator (16) contains the bacterial biomass to be recycled to the bottom part of the high mass-transfer bioreactor (6), through a pipeline system (17).

### BRIEF DESCRIPTION OF THE FIGURES

Figure 1. Flow-Scheme representing the biomilking process of the present invention.
1. Inlet for gas stream or streams containing methane and oxygen to the bioreactor
2. Outlet for gas stream from the bioreactor
3. Inlet for liquid stream to the bioreactor
4. Outlet for liquid outlet stream from the bioreactor
5. Liquid recirculation pipeline system
6. Gas-liquid mixing chamber
7. Taylor flow capillaries
8. Gas-liquid separator
9. Gas recirculation pipeline system
10. First solid-liquid separator
11. Liquid recirculation pipeline system
12. Outlet for the concentrated biomass stream
13. Inlet for water stream or lower salinity medium stream
14. Hypo-osmotic shock vessel (or tank)
15. Outlet for liquid stream
16. Second solid-liquid separator
17. Pipeline system for the biomass recirculation stream
18. Outlet for the osmolytes enrichment stream

### LIST OF REFERENCES

### • Patents

- WO2015058212A1: Microbial conversion of methane
- CN101314785A: Method for fermentation preparation of ectoine
- CN101173237A: Recycle of hyperosmotic waste liquor in halobacteria biological milking industrial process.
- EP0677044B1: (In vivo) method of obtaining cell components
- JP2012029679A: Method for producing compatible solute
- DE10114189B4: Method for obtaining useful products from organisms

### • Non-patent literature:

- Cantera, S., Lebrero, R., Rodriguez, S., Garcia-Encina, P.A., Muñoz, R., 2017. Ectoine bio-milking in methanotrophs: A step further towards methane-based bio-refineries into high added-value products. Chem. Eng. J. 328, 44-48. https://doi.org/10.1016/j.cej.2017.07.027
- Cantera, S., Muñoz, R., Lebrero, R., López, J.C., Rodriguez, Y., Garcia-Encina, P.A., 2018. Technologies for the bioconversion of methane into more valuable products. Curr. Opin. Biotechnol. 50, 128-135. https://doi.org/10.1016/j.copbio.2017.12.021
- Cantera, S., Sánchez-Andrea, I., Sadornil, L.J., Garcia-Encina, P.A., Stams, A.J.M., Muñoz, R., 2019, Novel haloalkaliphilic methanotrophic bacteria: An attempt for enhancing methane bio-refinery. J. Environ. Manag. 231, 1091-1099. https://doi.org/10.1016/j.jenvman.2018.11.017
- Carmona-Martinez, A.A., Marcos-Rodrigo, E., Bordel, S., Marín, D., Herrero-Lobo, R., Garcia-Encina, P.A., Muñoz, R., 2021. Elucidating the key environmental parameters during the production of ectoines from biogas by mixed methanotrophic consortia. J. Environ. Manage. 298. https://doi.org/10.1016/j.jenvman.2021.113462
- Pérez, V., Moltó, J.L., Lebrero, R., Muñoz, R., 2021. Ectoine Production from Biogas in Waste Treatment Facilities: A Techno-Economic and Sensitivity Analysis. ACS Sustain. Chem. Eng. 9, 17371-17380. https://doi.org/10.1021 /acssuschemeng.1 c06772
- Rodero, M.R., Herrero-Lobo, R., Pérez, V., Muñoz, R., 2022. Influence of operational conditions on the performance of biogas bioconversion into ectoines in pilot bubble column bioreactors. Bioresour. Technol. 358, 127398. https://doi.org/10.1016/j.biortech.2022.127398.
- Cattaneo C.R, Rodriguez Y, Rene E.R, Garcia-Depraect O, Munoz R (2022) Biogas bioconversion into poly(3-hydroxybutyrate) by a mixed microbial culture in a novel Taylor flow bioreactor. Waste Management. 150: 364-372. https://doi.org/10.1016/j.wasman.2022.07.017.

### EXAMPLE OF THE INVENTION

A 584 m³ Taylor-flow bioreactor was filled with 526 m³ of growth mineral medium containing 37 g/L of sodium nitrate, 83 g/L of sodium chloride and 0.4 g/L of micronutrients. The growth medium can be also brine wastewater supplemented with a trace metals solution containing at least cupper, iron, zinc, niquel, cobalt, molybdenum, manganese and boric acid. The bioreactor was inoculated with a consortium of extremophile methanotrophic bacteria.

A 100 Nm³/h methane laden gas stream consisting of biogas with a methane content of 60 %v/v was sparged into the bioreactor. A 431.9 Nm³/h oxygen laden gas stream consisting of air with an oxygen content of 21 %v/v was sparged into the bioreactor. The combined gas mixture, with a volumetric flow of 531.9 m³/h, was composed of 11.3 %v/v methane, 17.3 %v/v oxygen, 64.6 %v/v nitrogen and 7.5 %v/v carbon dioxide. The combined gas mixture presented a O₂:CH₄ ratio of 1.5.

A methane removal efficiency of 90 % was achieved, given the high gas-liquid mass transfer rate of Taylor-flow bioreactor, and therefore a 437.2 m³/h outlet gas stream was obtained, with a composition of 1.4 %v/v methane, 2.1 %v/v oxygen, 78.7 %v/v nitrogen and 17.8 %v/v carbon dioxide.

A 419 L/h liquid stream of fresh mineral medium, or alternatively of brine wastewater supplemented with a trace metals solution (containing at least cupper, iron, zinc, niquel, cobalt, molybdenum, manganese and boric acid) was continuously added to the bioreactor.

The bioreactor was operated at ambient pressure (1.5-2 bar) and ambient temperature (15-25 °C).

A 4,442 L/h liquid stream of bacterial culture containing 55.7 kg/h of biomass with an intracellular ectoine concentration of 50 mg ectoine / g biomass was continuously removed from the bioreactor and centrifuged to a biomass concentration of 200 g/L in a solid-liquid separator.

10 % of the liquid fraction from the separation was purged and the rest was recycled to the bioreactor.

Water was added to the concentrated biomass stream to a final biomass concentration of 33.4 g/L and then subjected to the hypo-osmotic shock in a stirred tank reactor where 70 % of the intracellular ectoine was released.

The biomilking stream was concentrated in a solid-liquid separator to a biomass concentration of 200 g/L.

25 % of the concentrated stream was purged from the system and the rest was recycled to the high mass-transfer bioreactor.

A 1,331.6 L/h liquid stream containing 2.64 kg ectoine was obtained.

## Claims

1. A method for converting methane into added-value products **characterized in that** a biological process using extremophile methanotrophic bacteria is carried out in a high-mass transfer bioreactor, wherein the method comprises:
a) cultivating an extremophile methanotrophic bacterial culture in a nutrient liquid solution comprising a content of salt of at least 0.05% w/w, more preferably between 1%w/w and 36%w/w, the nutrient liquid solution being one selected from the group consisting of: a synthetic solution of mineral salts, a waste stream and mixtures thereof;
b) injecting a gas flow containing methane and oxygen, in such a way that the flow of gas comprises between 1%v/v and 99%v/v of methane and between 1%v/v and 99%v/v of oxygen, the ratio of oxygen-to-methane being of at least 1.5 mol oxygen/mol methane;
c) extracting a fraction of the bacterial culture and subjecting to a concentration process of the biomass between 10 and 400 g biomass / L in a solid liquid separator;
d) subjecting the concentrated biomass to a hypo-osmotic shock during a time between 1 minute and 24 hours in an external tank by adding water to a salinity ranging from 0 %w/w to 6%w/w, lower than the one of the concentrated biomass, for partially releasing at least one osmoprotective osmolyte from the bacterial cells;
e) concentrating again the biomass after the hypo-osmotic shock to a concentration between 10 and 400 g biomass /L in a solid-liquid separator; and
f) extracting one or more osmoprotective osmolytes;
wherein the biomass is re-used by one of the methods selected from the group consisting of batch, fed-batch or continuous production cycles for obtaining a liquid stream containing osmolytes;
and wherein all or part of the used gas stream that leaves the bioreactor is recirculated by re-injecting said used gas stream into the bioreactor, separately or mixed with the gas flow containing methane and oxygen.

2. The method according to claim 1, wherein the high-mass transfer bioreactor is a Taylor-flow bioreactor.

3. The method according to any one of claims 1 or 2, wherein the extremophile methanotrophic bacteria is a haloalkalophilic methanotrophic bacteria or a halophilic methanotrophic bacteria.

4. The method according to any one of claims 1 to 3, wherein the gas flow is made of either one single gas stream or a combination of two or more gas streams that are injected separately, one methane-containing gas stream and one oxygen-containing gas stream.

5. The method according to claim 4, wherein the gas flow containing methane and oxygen is a by-product selected from the group consisting of natural gas, biogas and diluted methane emissions.

6. The method according to claim 5, wherein the by-product is biogas.

7. The method of claim 4, wherein the gas flow is a mixture of a methane-laden gas stream and an oxygen-laden gas stream, mixed in a single flow to be injected into the reactor.

8. The method according to claim 4, wherein the gas flow is a mixture of one methane-containing gas stream selected among biogas or natural gas, and one oxygen-containing gas stream selected among air or O₂ enriched air, the gas flow comprising an amount of between 1% and 20%v/v of methane in air of the total gas flow.

9. The method according to any one of claims 1 to 8, wherein the nutrient liquid solution of step a) is a single waste stream or a mixture of waste streams, supplemented or not with a solution of at least one trace metal.

10. The method according to claim 9, wherein, wherein at least one of the waste streams is brine wastewater supplemented with a solution of at least one trace metal.

11. The method according to any one of claims 9 or 10, wherein the trace metal is selected from the group of elements consisting of cupper, iron, zinc, nickel, cobalt, molybdenum, manganese, boric acid and any combination thereof.

12. The method according to any one of claims 1 to 11, wherein the nutrient liquid solution of step a) contains an amount of salt between 1% and 36% of the total amount of the flow.

13. The method according to any one of claims 1 to 12, wherein the osmoprotective osmolyte is selected from the group consisting of ectoine, hydroxyectoine or any derivative thereof.

14. The method according to any one of claims 1 to 13, wherein the hypo-osmotic shock is carried out during a period of time comprised between 1 minute and 24 hours.

15. The method according to any one of claims 1 to 14, wherein a liquid waste stream is produced in step c) that is recycled to the high-mass transfer bioreactor wherein the bacteria culture is cultivated;

16. The use of a high-mass transfer bioreactor in a biological process for converting methane into added-value products by using extremophile methanotrophic bacteria as the one defined in any one of claims 1 to 15.

17. The use of claim 16, wherein:
- the high-mass transfer bioreactor is a Taylor-flow bioreactor;
- the osmoprotective osmolytes are selected from the group consisting of ectoine, hydroxyectoine and any derivative thereof, and
- the biological process is a biomilking process.

## Amended claims

### Amended claims in accordance with Rule 137(2) EPC.

1. A method for converting methane into added-value products **characterized in that** a biological process using extremophile methanotrophic bacteria is carried out in a high-mass transfer bioreactor, wherein the method comprises:
a) cultivating an extremophile methanotrophic bacterial culture in a nutrient liquid solution comprising a content of salt of at least 0.05% w/w, more preferably between 1%w/w and 36%w/w, the nutrient liquid solution being one selected from the group consisting of: a synthetic solution of mineral salts, a waste stream and mixtures thereof;
b) injecting a gas flow containing methane and oxygen, in such a way that the flow of gas comprises between 1%v/v and 99%v/v of methane and between 1%v/v and 99%v/v of oxygen, the ratio of oxygen-to-methane being of at least 1.5 mol oxygen/mol methane;
c) extracting a fraction of the bacterial culture and subjecting to a concentration process of the biomass between 10 and 400 g biomass / L in a solid liquid separator;
d) subjecting the concentrated biomass to a hypo-osmotic shock during a time between 1 minute and 24 hours in an external tank by adding water to a salinity ranging from 0 %w/w to 6%w/w, lower than the one of the concentrated biomass, for partially releasing at least one osmoprotective osmolyte from the bacterial cells;
e) concentrating again the biomass after the hypo-osmotic shock to a concentration between 10 and 400 g biomass /L in a solid-liquid separator; and
f) extracting one or more osmoprotective osmolytes;
wherein the biomass is re-used by one of the methods selected from the group consisting of batch, fed-batch or continuous production cycles for obtaining a liquid stream containing osmolytes;
wherein all or part of the used gas stream that leaves the bioreactor is recirculated by re-injecting said used gas stream into the bioreactor, separately or mixed with the gas flow containing methane and oxygen; and
wherein the high-mass transfer bioreactor is a Taylor-flow bioreactor.

2. The method according to claim 1, wherein the extremophile methanotrophic bacteria is a haloalkalophilic methanotrophic bacteria or a halophilic methanotrophic bacteria.

3. The method according to any one of claims 1 or 2, wherein the gas flow is made of either one single gas stream or a combination of two or more gas streams that are injected separately, one methane-containing gas stream and one oxygen-containing gas stream.

4. The method according to claim 3, wherein the gas flow containing methane and oxygen is a by-product selected from the group consisting of natural gas, biogas and diluted methane emissions.

5. The method according to claim 4, wherein the by-product is biogas.

6. The method of claim 3, wherein the gas flow is a mixture of a methane-laden gas stream and an oxygen-laden gas stream, mixed in a single flow to be injected into the reactor.

7. The method according to claim 3, wherein the gas flow is a mixture of one methane-containing gas stream selected among biogas or natural gas, and one oxygen-containing gas stream selected among air or O₂ enriched air, the gas flow comprising an amount of between 1% and 20%v/v of methane in air of the total gas flow.

8. The method according to any one of claims 1 to 7, wherein the nutrient liquid solution of step a) is a single waste stream or a mixture of waste streams, supplemented or not with a solution of at least one trace metal.

9. The method according to claim 8, wherein, wherein at least one of the waste streams is brine wastewater supplemented with a solution of at least one trace metal.

10. The method according to any one of claims 8 or 9, wherein the trace metal is selected from the group of elements consisting of cupper, iron, zinc, nickel, cobalt, molybdenum, manganese, boric acid and any combination thereof.

11. The method according to any one of claims 1 to 10, wherein the nutrient liquid solution of step a) contains an amount of salt between 1% and 36% of the total amount of the flow.

12. The method according to any one of claims 1 to 11, wherein the osmoprotective osmolyte is selected from the group consisting of ectoine, hydroxyectoine or any derivative thereof.

13. The method according to any one of claims 1 to 12, wherein the hypo-osmotic shock is carried out during a period of time comprised between 1 minute and 24 hours.

14. The method according to any one of claims 1 to 13, wherein a liquid waste stream is produced in step c) that is recycled to the high-mass transfer bioreactor wherein the bacteria culture is cultivated;

15. The use of a high-mass transfer bioreactor in a biological process for converting methane into added-value products by using extremophile methanotrophic bacteria as the one defined in any one of claims 1 to 14.

16. The use of claim 15, wherein:
- the high-mass transfer bioreactor is a Taylor-flow bioreactor;
- the osmoprotective osmolytes are selected from the group consisting of ectoine, hydroxyectoine and any derivative thereof, and
- the biological process is a biomilking process.
